# EUROPEAN PATENT APPLICATION

(11) **EP 4 623 872 A1**
(43) Date of publication of application: **01.10.2025**
(21) Application number: 24209208.8
(22) Date of filing: 28.10.2024
(51) Int. Cl.: A61F 2/74, A61F 2/50

(54) **FLOW DIFFUSER VALVE FOR VARIABLE HYDRAULIC RESISTANCE**

(30) Priority: 28.03.2024 US 202463571083 P
(71) Applicant: Blatchford Products Limited, Basingstoke, Hampshire RG24 8PZ (GB)
(72) Inventor: Preece, Adam Clive, Wolverhampton WV3 8EF (GB)
(74) Representative: Hepworth Browne

(57) **Abstract**

Embodiments disclosed herein provides, a flow diffuser valve (100) for variable hydraulic resistance in a prosthetic or orthotic device. The flow diffuser valve (100) includes a sleeve (1) component comprising a plurality of set of holes (3) distributed throughout a surface of the sleeve (1) component. Further, the flow diffuser valve (100) includes a spool (2) component located inside the sleeve (1) component, wherein a rotational or an axial movement of the spool (2) component relative to the sleeve (1) component generates a dynamic variation in a number of open holes of the plurality of set of holes (3). The number of open holes limits a flow of fluid passing through the flow diffuser valve (100) to achieve the variable hydraulic resistance control at the flow diffuser valve (100).

## Description

### FIELD OF INVENTION

The present disclosure relates to medical devices and, more particularly, to a lamina flow diffuser valve designed to achieve variable hydraulic resistance in prosthetic and orthotic devices.

### BACKGROUND

Prosthetics serve as artificial replacements for body parts such as fingers, hands, arms, and legs. Typically, they are employed to replace body parts lost either from injury or at birth. Limb amputations can occur as a result of traumatic events or conditions such as diabetes, dysvascular disease, or tumors. A limb prosthesis constitutes an artificial device that is designed to replace the lost limb, with customization tailored to meet the individual's specific requirements. It is created to mimic the function of the missing limb, but its use requires a significant amount of energy when compared to that of an able-bodied individual. This energy discrepancy occurs because the current limb prostheses suffer from significant energy loss during use by patients.

The power behind natural limb joints emanates from the actions of muscles that generate active force through contraction and also provide variable stiffness or resistance. However, duplicating muscle contraction in leg prostheses has been unfeasible due to the weight and bulk required to imitate the function of natural limb joints. Consequently, conventional systems has focused on implementing stiffness or resistance to rotation of the knee joint. This typically involves switching the knee joint between one of two modes, locked or free to rotate. During the stance phase of the gait cycle, the knee joint is locked, while during the swing phase, it is free to rotate. The stance phase occurs when the foot of the prosthesis is on the ground, while the swing phase refers to the period when the foot of the prosthesis is off the ground.

Hydraulic prosthetic and orthotic devices, particularly those operating under high load conditions - such as knee units when descending stairs - rely on hydraulic control valves to control the flow of hydraulic fluid through the valve and generate the pressure which provides the resistance required. Such valves achieve this by moving a component (usually a spool) to restrict the hydraulic flow by varying the available cross-sectional area through which the fluid must flow.

Under load at maximum resistance settings, the control valves produce considerable pressure. For these high resistance settings metering feature/s are adjusted to show a very small cross sectional area to the flow, resulting in high fluid velocities often exceeding 100m/s and a turbulent flow regime. This, in turn, can lead to unpleasant local noise that users tend to find unfavorable. Furthermore, when a valve has multiple metering features, attaining high positional accuracy between them typically necessitates meticulous grinding and even axial shimming of components to maintain axial alignment.

Several hydraulic mechanisms have been incorporated into lower extremity prosthetics with the aim of facilitating walking and enabling patients to adjust the resistance within a pneumatic or hydraulic cylinder during swing phase. This allows for greater control of knee joint rotation across a range of walking speeds beyond that achievable with traditional pneumatic or friction dampers. While recent hydraulic devices are considered to be enhanced, they remain intricate and expensive to produce. Moreover, conventional hydraulic devices necessitate a high degree of precision in their production.

Thus, it is desired to address the above-mentioned disadvantages, issues, or other shortcomings or at least provide a useful alternative.

### OBJECT OF INVENTION

The principal object of the embodiments herein is to provide a flow diffuser valve to achieve variable hydraulic resistance in prosthetic and orthotic devices.

Yet another object of the embodiments herein is to provide the flow diffuser valve that employs a plurality of micro holes to restrict the flow and attain a variable hydraulic resistance, predominantly under the lamina flow regime.

Yet another object of the embodiments herein is to provide the flow diffuser valve with adaptable hydraulic resistance, while keeping the generated noise at minimal levels. This is accomplished by implementing the plurality of micro holes within a sleeve portion of the flow diffuser valve.

### SUMMARY

Embodiments disclosed herein provides, a flow diffuser valve for variable hydraulic resistance in a prosthetic or orthotic device. The flow diffuser valve includes a sleeve component comprising a plurality of micro holes distributed throughout a surface of the sleeve component. Further, the flow diffuser valve includes a spool component located inside the sleeve component, wherein a rotary movement or an axial movement of the spool component relative to the sleeve component generates a dynamic variation in a number of open holes of the plurality of micro holes. The number of open holes limits a flow of fluid passing through the flow diffuser valve to achieve the variable hydraulic resistance control at the flow diffuser valve.

In an embodiment, the flow of the fluid remains completely lamina within the open holes, resulting in a pressure drop as the fluid is compelled through, resulting in low levels of noise generated by the flow diffuser valve.

In an embodiment, a diameter of each hole of the set of holes is 0.25 mm or less, and the set of holes is 15 or more

In an embodiment, each set of holes of the plurality of set of holes of the flow diffuser valve are separated by 120 degree.

In an embodiment, the plurality of set of holes are configured to direct the flow of fluid in a predetermined pattern to achieve variation in the hydraulic resistance.

In an embodiment, the spool component is driven by a control element, wherein the spool interfaces with the control element and allows the transfer of a load from a driving device to the spool, wherein the spool comprises a central bore that receives the control element for controlling the rotational movement or axial movement of the spool component.

In an embodiment, the sleeve component comprises flow metering features, wherein the sleeve houses the spool and a plurality of hydraulic seals.

In an embodiment, the flow diffuser valve further comprises a housing component configured to enclose the sleeve component and the spool component, and wherein the housing component comprises inlet and outlet ports for the flow of fluid through the flow diffuser valve. In an embodiment, the inlet and outlet ports are configured to connect to a hydraulic circuit of the prosthetic device.

In an embodiment, the flow diffuser valve is configured to provide variable hydraulic resistance to the flow of fluid through the hydraulic circuit of the prosthetic or orthotic device, and wherein the variable hydraulic resistance is adjustable based on the position of the spool component within the sleeve component.

These and other aspects of the embodiments herein will be better appreciated and understood when considered in conjunction with the following description and the accompanying drawings. It should be understood, however, that the following descriptions, while indicating preferred embodiments and numerous specific details thereof, are given by way of illustration and not of limitation. Many changes and modifications may be made within the scope of the embodiments herein without departing from the scope thereof, and the embodiments herein include all such modifications.

### BRIEF DESCRIPTION OF FIGURES

These and other features, aspects, and advantages of the present disclosure are illustrated in the accompanying drawings, throughout which like reference letters indicate corresponding parts in the various figures. The embodiments herein will be better understood from the following description with reference to the drawings, in which:

FIGS. 1A-1G illustrates different perspective views of flow diffuser valve for variable hydraulic resistance, according to the embodiments as disclosed herein;

FIGS. 2A-2C illustrates sectional views of the flow diffuser valve for the variable hydraulic resistance, according to the embodiments as disclosed herein; and

FIGS. 3A-3F illustrates different perspective views of a spool used in the flow diffuser valve for the variable hydraulic resistance, according to the embodiments as disclosed herein.

It may be noted that to the extent possible, like reference numerals have been used to represent like elements in the drawing. Further, those of ordinary skill in the art will appreciate that elements in the drawing are illustrated for simplicity and may not have been necessarily drawn to scale. For example, the dimension of some of the elements in the drawing may be exaggerated relative to other elements to help to improve the understanding of aspects of the invention. Furthermore, the elements may have been represented in the drawing by conventional symbols, and the drawings may show only those specific details that are pertinent to the understanding the embodiments of the invention so as not to obscure the drawing with details that will be readily apparent to those of ordinary skill in the art having benefit of the description herein.

### DETAILED DESCRIPTION OF INVENTION

The embodiments herein and the various features and advantageous details thereof are explained more fully with reference to the non-limiting embodiments that are illustrated in the accompanying drawings and detailed in the following description. Descriptions of well-known components and processing techniques are omitted so as to not unnecessarily obscure the embodiments herein. Also, the various embodiments described herein are not necessarily mutually exclusive, as some embodiments can be combined with one or more other embodiments to form new embodiments. The term "or" as used herein, refers to a non-exclusive or, unless otherwise indicated. The examples used herein are intended merely to facilitate an understanding of ways in which the embodiments herein can be practiced and to further enable those skilled in the art to practice the embodiments herein. Accordingly, the examples should not be construed as limiting the scope of the embodiments herein.

Embodiments disclosed herein provides, a flow diffuser valve for variable hydraulic resistance in a prosthetic or orthotic device. The flow diffuser valve includes a sleeve component comprising a plurality of micro holes distributed throughout a surface of the sleeve component. Further, the flow diffuser valve includes a spool component located inside the sleeve component, wherein a rotational movement or an axial movement of the spool component relative to the sleeve component generates a dynamic variation in a number of open holes of the plurality of micro holes. The number of open holes limits a flow of fluid passing through the flow diffuser valve to achieve the variable hydraulic resistance control at the flow diffuser valve.

In an embodiment, the flow of the fluid remains completely lamina within the open holes, resulting in a pressure drop as the fluid is compelled through, resulting in low levels of noise generated by the flow diffuser valve.

In an embodiment, a diameter of each hole of the set of holes is 0.25 mm or less, and the set of holes is 15 or more

In an embodiment, each set of holes of the plurality of set of holes of the flow diffuser valve are separated by 120 degree.

In an embodiment, the plurality of set of holes are configured to direct the flow of fluid in a predetermined pattern to achieve variation in the hydraulic resistance.

In an embodiment, the spool component is driven by a control element, wherein the spool interfaces with the control element and allows the transfer of a load from a driving device to the spool, wherein the spool comprises a central bore that receives the control element for controlling the rotational movement or axial movement of the spool component.

In an embodiment, the sleeve component comprises flow metering features, wherein the sleeve houses the spool and a plurality of hydraulic seals.

In an embodiment, the flow diffuser valve further comprises a housing component configured to enclose the sleeve component and the spool component, and wherein the housing component comprises inlet and outlet ports for the flow of fluid through the flow diffuser valve. In an embodiment, the inlet and outlet ports are configured to connect to a hydraulic circuit of the prosthetic device.

In an embodiment, the flow diffuser valve is configured to provide variable hydraulic resistance to the flow of fluid through the hydraulic circuit of the prosthetic or orthotic device, and wherein the variable hydraulic resistance is adjustable based on the position of the spool component within the sleeve component.

This particular design has the versatility to be employed in various configurations, without being constrained to the examples provided. For instance, a single valve can be utilized at a specific juncture in the hydraulic circuit to restrict the flow and create an adjustable pressure drop. Alternatively, two valves can be implemented at distinct points in the hydraulic circuit to limit the flow, producing variable pressure drops, commonly linked with opposing directions of movement of a hydraulic piston or rotor. Another possibility is the use of a single valve that restricts the hydraulic flow in both directions of travel for the hydraulic piston or rotor in a hydraulic circuit, generating a variable pressure drop. Typically, this setup would comprise two sets of metering components on the flow diffuser valve and entail directing two sets of drillways into and out of the flow diffuser valve.

In conventional method, under load the control valves produce considerable pressure. To attain higher resistances, metering feature/s are adjusted to restrict the flow by leaving a small cross sectional area for the flow to travel through, resulting in high fluid velocities typically exceeding 100m/s and a turbulent flow regime. This, in turn, can lead to unpleasant local noise that users tend to find unfavorable. Furthermore, when a valve has multiple metering features, attaining high positional accuracy between them typically necessitates meticulous grinding and even axial shimming of components to maintain axial alignment.

In conventional method, several hydraulic devices have been incorporated into lower extremity prosthetic and orthotic devices with the aim of facilitating walking and enabling patients to adjust the resistance within a pneumatic or hydraulic cylinder during swing phase. This allows for greater control of knee joint rotation across a range of walking speeds beyond that achievable with traditional pneumatic or friction dampers. While recent hydraulic devices are considered to be enhanced, they remain intricate and expensive to produce. Moreover, conventional hydraulic devices necessitate a high degree of precision in their production.

Unlike the conventional method, the present disclosure utilizes the plurality of set of holes in the sleeve portion of the flow diffuser valve to limit the flow and achieve variable hydraulic resistance with a predominantly lamina flow regime which results in low level noise output. The flow diffuser valve does not need shimming to achieve valve's axial alignment.

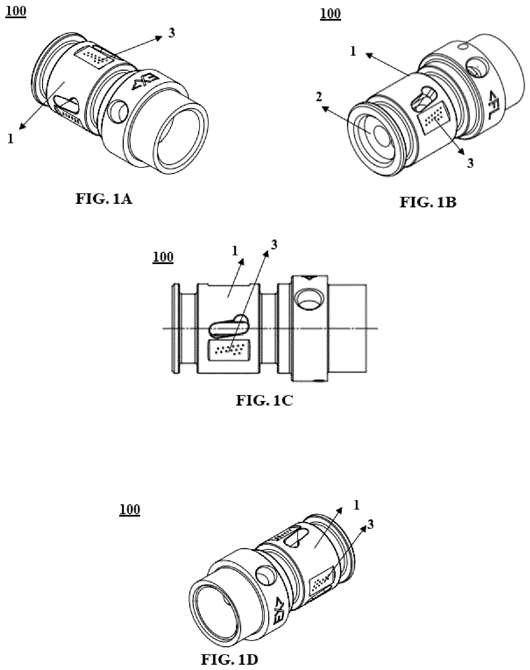

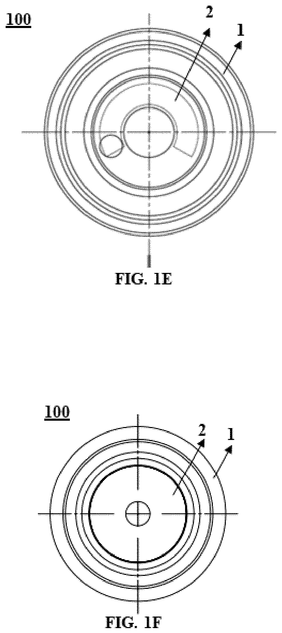

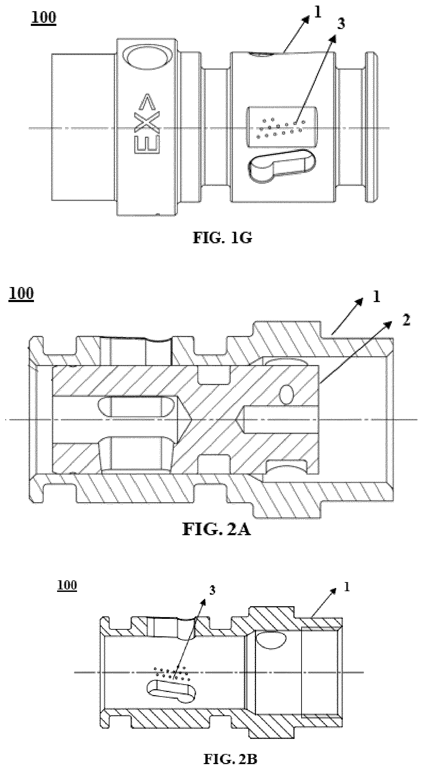

Referring now to the drawings, and more particularly to FIGS. 1A through 3F where similar reference characters denote corresponding features consistently throughout the figures, there are shown preferred embodiments.

FIGS. 1A-1G illustrates different perspective views of flow diffuser valve (100) for variable hydraulic resistance, according to the embodiments as disclosed herein. As shown in FIG. 1A the flow diffuser valve (100) to achieve variable hydraulic resistance includes a sleeve (1) component which includes a plurality of set of holes (3), and a spool (2) component that rotates vis-à-vis the sleeve (1) to enable axial movement. The axial movement leads to a dynamic variation in the number of open set of holes (3), thereby facilitating the flow of fluid through the valve (100).

In an embodiment, the sleeve (1) component includes three sets of holes (3), each separated by a fixed distance (e.g. 120 degrees) around the circumference of the sleeve (1). The flow diffuser valve (100) uses the plurality of set of holes (3) to limit the flow and achieve a variable hydraulic resistance with a predominantly lamina flow regime, which results a regulation valve with exceptionally low noise levels, even for the high resistances the valve (100) delivers. Typically the sleeve (1) has 15 or more set of holes (3) of diameter 0.25mm or less. The flow diffuser valve (100) as shown in the FIG. 1A has a total of 42 holes (3) with each aperture having diameter 0.25mm and shared between three banks. The banks are separated certain distance (e.g. 120 degree) around the circumference of the sleeve (1). The spool (2) component which rotates and moves relative to the sleeve (1) provides openings in the form of 3 slots which will vary the number of open set of holes (3) that are seen by the fluid and which the fluid will travel through.

In an embodiment, the spool is drive by a motor, the rotational movement of the motor relative to the sleeve (1) drives the spool (2) to regulate the pressure drop across the valve (100) based on the specific fluid and flow rate. This is achieved through the manipulation of the number of set of holes (3) exposed to the fluid. By increasing the number of open holes, the pressure drop decreases as the fluid passes through the valve (100). Conversely, reducing the number of open apertures increases the pressure drop during fluid passage.

The use of the set of holes (3) was a deliberate choice, as it permits exceptional resistance control while maintaining predominantly laminar flow, even at the highest resistance settings and low Reynolds numbers. Consequently, the noise levels associated with the valve's operation remain very low.

In an embodiment, the design featuring the set of holes (3) within the sleeve (1) section has been chosen to regulate and direct fluid flow into a laminar flow regime, where the majority of pressure drop is caused by viscous fluid drag. This results in the valve (100) generating low levels of turbulence and negligible amounts of noise, courtesy of the high laminar viscous drag achieved.

In an embodiment, the flow diffuser valve (100) further comprises a housing component configured to enclose the sleeve (1) component and the spool (2) component, and wherein the housing component comprises inlet and outlet ports for the flow of fluid through the flow diffuser valve. In an embodiment, the inlet and outlet ports are configured to connect to the hydraulic circuit of the prosthetic or orthotic device.

In an embodiment, the flow diffuser valve (100) is configured to provide variable hydraulic resistance to the flow of fluid through the hydraulic circuit of the prosthetic or orthotic device, and wherein the variable hydraulic resistance is adjustable based on the position of the spool (2) component within the sleeve (1) component.

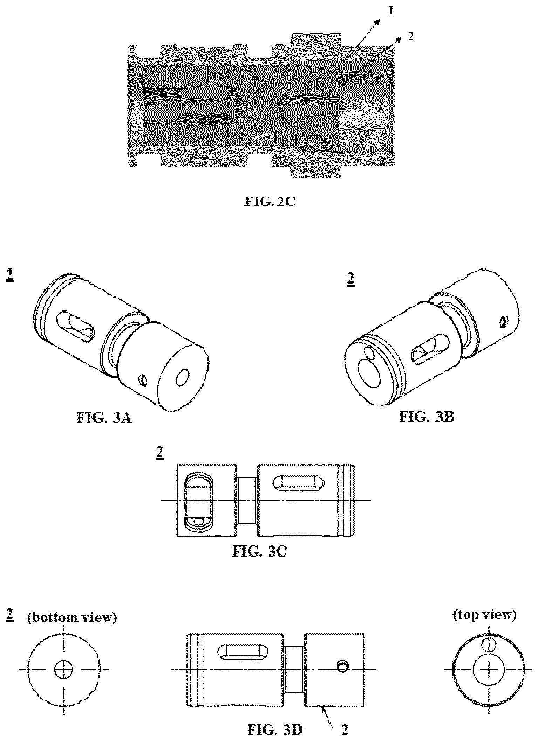

FIGS. 2A-2C illustrates sectional views of the flow diffuser valve (100) for variable hydraulic resistance, according to the embodiments as disclosed herein.

The flow diffuser valve (100) can be configured in various ways to suit different applications. In an embodiment, the configuration includes using a single valve (100) in the hydraulic circuit to regulate flow and create a variable pressure drop.

In another embodiment, two valves (100) can be employed at two different points in the hydraulic circuit to limit the flow and produce variable pressure drops. This configuration is typically used when the hydraulic piston or rotor moves in opposite directions.

In another embodiment, a single valve (100) is used to limit flow in both directions of travel for the piston or rotor in the hydraulic circuit. This configuration typically includes two sets of metering features on the valve (100), which are connected to two sets of drill ways to effectively control pressure drops.

In an embodiment, the sleeve (1) component comprises flow metering features, wherein the sleeve (1) houses the spool (2) and a plurality of hydraulic seals. The hydraulic seals in flow diffuser valve (100) are critical components that ensure the efficient and leak-proof operation of hydraulic systems. The hydraulic seals prevents leaks, maintain pressure, reduce wear and tear and ensure smooth operation.

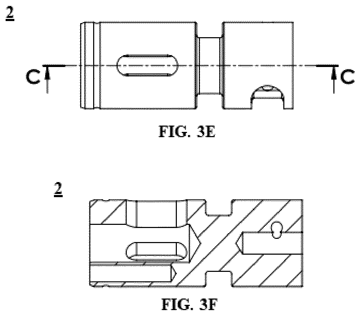

FIGS. 3A-3F illustrates different perspective views of the spool (2) used in the flow diffuser valve (100) for variable hydraulic resistance, according to the embodiments as disclosed herein. The spool (2) component which rotates and moves relative to the sleeve (1) provides openings in the form of three slots which will vary the number of open set of holes (3) that are seen by the fluid and which the fluid will travel through.

In an embodiment, the flow diffuser valve (100) exhibits a remarkable level of noise reduction in comparison to conventional valves. This is due to the valve's flow regime, which is lamina and consequently yields very low Reynolds numbers. As per user feedback, quieter valves (100) would enhance the overall product experience and subsequently increase user satisfaction levels.

In an embodiment, the flow diffuser valve's set of holes (3), which serve as flow metering features, require less precise positioning compared to conventional flow metering valves with multiple flow metering features. The design of the flow diffuser valve (100) allows for the flow metering set of holes (3) to have lower positional accuracy without significantly impacting the valve's (100) functionality. Furthermore, the flow diffuser valve (100) eliminates the need for shimming or grinding to achieve axial alignment.

In an embodiment, the valve (100) exhibits a reduced tendency to induce fluid cavitation. The flow diffuser valve (100) is endowed with extensive lamina fluid friction, which typically accounts for the majority of the pressure drop across the valve (100) during normal operation. Consequently, fluid velocities are diminished, and the local pressure downstream of the flow metering components is elevated, thereby mitigating the probability of cavitation occurrence. Specifically, the influence of the Venturi effect, the local minimum pressure downstream of the flow metering features, and the subsequent pressure recovery from the valve (100) are all substantially reduced.

In an embodiment, the set of holes (3) pattern of the lamina flow diffuser valve (100) is customizable to specifications (including the number of apertures, spacing between apertures, and individual hole sizes), thus allowing for a customized resistance profile as the valve (100) undergoes its stroke.

The foregoing description of the specific embodiments will so fully reveal the general nature of the embodiments herein that others can, by applying current knowledge, readily modify and/or adapt for various applications such specific embodiments without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation. Therefore, while the embodiments herein have been described in terms of preferred embodiments, those skilled in the art will recognize that the embodiments herein can be practiced with modification within the scope of the embodiments as described herein.

## Claims

1. A flow diffuser valve (100) for variable hydraulic resistance in a prosthetic or orthotic device comprising:
a sleeve (1) component comprising a plurality of set of holes (3) distributed throughout a surface of the sleeve (1) component; and
a spool (2) component located inside the sleeve (1) component, wherein a rotational movement or an axial movement of the spool (2) component relative to the sleeve (1) component generates a dynamic variation in a number of open holes of the plurality of set of holes (3),
wherein the number of open holes limits a flow of fluid passing through the flow diffuser valve (100) to achieve the variable hydraulic resistance control at the flow diffuser valve (100).

2. The flow diffuser valve (100) as claimed in claim 1, wherein the flow of the fluid remains completely lamina within the open holes, resulting in a pressure drop as the fluid is compelled through, resulting in low levels of noise generated by the flow diffuser valve (100).

3. The flow diffuser valve (100) as claimed in claim 1 or claim 2, wherein a diameter of each hole of the set of holes (3) is 0.25 mm or less.

4. The flow diffuser valve (100) as claimed in any preceding claim, wherein the set of holes (3) is 15 or more.

5. The flow diffuser valve (100) as claimed in any preceding claim, wherein each set of holes (3) of the plurality of set of holes (3) are separated by 120 degree.

6. The flow diffuser valve (100) as claimed in any preceding claim, wherein the plurality of set of holes (3) are configured to direct the flow of fluid in a predetermined pattern to achieve variation in the hydraulic resistance.

7. The flow diffuser valve (100) as claimed in any preceding claim, wherein the spool (2) component is driven by a control element, wherein the spool interfaces with the control element and allows the transfer of a load from a driving device to the spool (2), wherein the spool (2) comprises a central bore that receives the control element for controlling the rotational movement or axial movement of the spool (2) component.

8. The flow diffuser valve (100) as claimed in any preceding claim, wherein the sleeve (1) component comprises flow metering features, wherein the sleeve (1) houses the spool (2) and a plurality of hydraulic seals.

9. The flow diffuser valve (100) as claimed in any preceding claim, wherein the flow diffuser valve (100) further comprises a housing component configured to enclose the sleeve (1) component and the spool (2) component, and wherein the housing component comprises inlet and outlet ports for the flow of fluid through the flow diffuser valve (100).

10. The flow diffuser valve (100) as claimed in claim 9, wherein the inlet and outlet ports are configured to connect to a hydraulic circuit of the prosthetic device;

11. The flow diffuser valve (100) as claimed in any preceding claim, wherein the flow diffuser valve (100) is configured to provide variable hydraulic resistance to the flow of fluid through the hydraulic circuit of the prosthetic device, and wherein the variable hydraulic resistance is adjustable based on the position of the spool (2) component within the sleeve (1) component.
